# EUROPÄISCHE PATENTANMELDUNG

(11) **EP 3 753 501 A1**
(43) Veröffentlichungstag der Anmeldung: **23.12.2020**
(21) Anmeldenummer: 19181620.6
(22) Anmeldetag: 21.06.2019
(51) Int. Cl.: A61B 17/15, A61B 17/17

(54) **SCHABLONE UND SETS FÜR EINE DOMOSTEOTOMIE**

(71) Anmelder: Medartis Holding AG, 4057 Basel (CH)
(72) Erfinder: Knupp, Markus, 4103 Bottmingen (CH); Schumacher, Ralf, 4411 Seltisberg (CH); Rüegg, Jasmine, 4051 Basel (CH)
(74) Vertreter: Hepp Wenger Ryffel AG

(57) **Zusammenfassung**

Die Erfindung betrifft eine Schablone (1) für eine Domosteotomie. Die Schablone (1) weist einen Basisteil (2) mit zumindest einer Aufnahme (16) für ein Knochenfixierungselement auf. Der Basisteil weist zudem zumindest eine Fixierungsführung (3) zur Festlegung einer Plattenlochposition (29) auf. Die Schablone (2) weist einen Korrekturteil (9) auf, wobei der Korrekturteil (9) zumindest eine Korrekturführung (10) zur Festlegung einer weiteren Plattenlochposition (29) an einem zweiten Knochenteil (27) aufweist. Zudem weist die Schablone (2) einen Osteotomieteil (4) mit zumindest einer Osteotomieführung (6) für eine Domosteotomie zwischen dem ersten und dem zweiten Knochenteil (26, 27) auf.

## Beschreibung

Die vorliegende Erfindung betrifft eine Schablone für eine Domosteotomie. Ausserdem betrifft die vorliegende Erfindung verschiedene Sets, die diese Schablone enthalten. Weiterhin betrifft die Erfindung ein Verfahren zum Vorbereiten einer Fixation durch eine Osteosynthese.

Ein Anwendungsgebiet für eine Osteotomie ist eine Fehlstellung des oberen Sprunggelenks. Beispielsweise kann die Fehlstellung aufgrund von Fehl- und/oder Überbelastung entstehen. Weitere Ursachen sind fehlverheilte Knochenbrüche, Wachstumsstörungen, Knorpelschäden, insbesondere Arthrose mit Befall in der Knochengrenzzone und Fussdeformitäten. Durch die Fehlstellung können an den Beinen Achsabweichungen im Varus- (O-Konfiguration) oder Valgus-Sinne (X-Konfiguration) vorliegen.

Derartige Defekte können durch eine Osteotomie im oberen Sprunggelenk korrigiert werden. Bekannte Osteotomien sind aufklappend oder zuklappend. D. h., dass ein Keil aus dem Knochen herausgeschnitten wird und der Knochen dann nach innen oder nach aussen geklappt wird. Anschliessend werden die Knochenteile mit einer Osteosyntheseplatte zueinander fixiert und können ausheilen. Nachteilig an der bekannten Osteotomie ist, dass der Korrekturwinkel stark abhängig ist von chirurgischen Fähigkeiten des Operateurs. So können Abweichungen bei der Grösse des Keils, der herausgeschnitten wird, entstehen.

Ein weiteres Problem ist, dass die Trennflächen nicht zueinander passen. In der Folge können Druckspitzen im Gelenk entstehen und die Heilungsphase wird verlängert.

Das Gebrauchsmuster DE 296 12 306 zeigt eine Alternative zur Keilosteotomie. Das Dokument zeigt eine Bohrlehre für eine Domosteotomie. Die Bohrlehre weist auf einem Halbkreissektor dicht nebeneinanderliegende Bohrstiftführungen auf. Durch die Bohrstiftführungen kann jeweils ein Loch entlang des Halbkreissektors gebohrt werden. Anschliessend wird mittels Meisselschlägen der Knochen definitiv durchtrennt. Dadurch kann eine runde Trennlinie erzeugt werden. Zwar ermöglicht DE 296 12 306 eine Domosteotomie, aber der Korrekturwinkel, in welchem die Knochen anschliessend zueinander fixiert werden, ist weiterhin ungenau. Ausserdem ist die Operation mit der vorgeschlagenen Bohrlehre aufwendig.

US 6,190,390 zeigt ein Instrumentensystem und ein Verfahren zur Erstellung einer Domosteotomie. Das System weist eine Bohrlehre und einen Verlängerungsbalken auf. Die Bohrlehre weist eine Mehrzahl an Löchern auf, die entlang eines Bogens angeordnet sind. Die Bohrlehre wird mittels einer Verankerungsbohrung im Zentrum der Bohrlehre fixiert. Auch US 6, 190, 390 hat den Nachteil, dass ein Korrekturwinkel nicht präzise eingestellt werden kann und die nachfolgende Fixierung ungenau ist.

Es ist daher die Aufgabe der Erfindung, eine Osteotomie zu ermöglichen, die eine präzise Korrektur der Fehlstellung erlaubt. Insbesondere ist es die Aufgabe der Erfindung, die Nachteile des Standes der Technik zu überwinden.

In einer ersten Variante der Erfindung wird eine Schablone für eine Domosteotomie bereitgestellt. Die Schablone weist einen Basisteil mit zumindest einer Aufnahme für ein Knochenfixierungselement auf. Das Knochenfixierungselement ist zum Fixieren des Basisteils an einem ersten Knochenteil geeignet. Der Basisteil weist zumindest eine Fixierungsführung zur Festlegung einer Plattenlochposition auf. Die Schablone weist einen Korrekturteil auf, wobei der Korrekturteil zumindest eine Korrekturführung zur Festlegung einer weiteren Plattenlochposition an einem zweiten Knochenteil aufweist. Zudem weist die Schablone einen Osteotomieteil mit zumindest einer Osteotomieführung für eine Domosteotomie zwischen dem ersten und dem zweiten Knochenteil auf.

Mit der vorgeschlagenen Schablone kann eine Domosteotomie durchgeführt werden und gleichzeitig vor der Durchführung der Osteotomie die Position für eine Osteosyntheseplatte festgelegt werden. In der Folge kann eine Position, in der die Platte fixiert werden soll im Vornerein präzise vorgeben werden.

Das Knochenfixierungselement kann ein Kirschnerdraht ("K-Wire") sein. Die Aufnahme ist insbesondere zu Führung eines Kirschnerdrahtes geeignet sein. Die Aufnahme ist bevorzugt ein Durchgangsloch.

Das Grundprinzip einer Domosteotomie (auch bekannt als Pendel- oder Scheibenwischerosteotomie) beruht darauf, dass ein Knochen entlang von domförmigen Trennungsflächen (sog. Osteotomieflächen) in zwei Teile getrennt wird. Die Trennungsflächen können dann intra-operativ ohne Translationsverschiebung der Knochen justiert werden, indem die Knochenteile entlang der Trennungsflächen zueinander verdreht werden, sodass eine Fehlstellung korrigiert wird.

Als "-teil" soll hier und im verfolgenden etwas verstanden werden, das mit etwas anderem zusammen ein Ganzes bildet. So kann beispielsweise der Knochen, welcher in der Osteotomie getrennt wird, einen ersten und einen zweiten Teil aufweisen. Ein "-teil" kann beweglich mit einem oder mehreren anderen "-teilen" verbunden sein. Beispielsweise kann der Osteotomieteil oder der Korrekturteil beweglich mit dem Basisteil verbunden sein.

Die Fixierungsführung und/oder die Osteotomieführung und/oder die Korrekturführung sind bevorzugt Bohrführungen. Die Bohrführungen haben einen Durchmesser und eine Tiefe. Eine Achse der Bohrführungen ist bevorzugt senkrecht zu einer einem Benutzer zugewandten Oberfläche der Schablone. Die Achsen der Bohrführungen können parallel zueinander sein. In alternativen Ausführungsformen können die Achsen die Bohrführungen zueinander geneigt sein.

In einer bevorzugten Ausführungsform sind die Fixierungsführung und die Korrekturführung derart angeordnet, dass die damit erzeugten Plattenlochpositionen die Korrektur einer Knochenfehlstellung erlauben. Insbesondere weist die Schablone zwei, drei oder mehr Fixierungsführungen auf. Die Schablone kann zwei, drei oder mehr Korrekturführungen aufweisen.

Dadurch kann ein Korrekturwinkel, um den die Fehlstellung korrigiert werden soll, im Vornherein präzise am Knochen festgelegt werden. Vor der Operation kann dieser Korrekturwinkel beispielsweise mithilfe von Röntgenbildern festgelegt werden und dann durch die Festlegung der Plattenlochposition auf beiden Seiten der Osteotomie vorbestimmt werden. In einer Variante ist die Schablone wiederverwendbar.

Zwei oder mehr Fixierungsführungen können entlang einer imaginären, geraden Fixierlinie angeordnet sein. Zwei oder mehr Korrekturführungen können entlang einer imaginären, geraden Fixierlinie angeordnet sein, wobei ein vorbestimmter Winkel zwischen der Fixier- und der Korrekturlinie liegt.

Der vorbestimmte Winkel gibt einen Korrekturwinkel zwischen dem ersten Knochenteil und dem zweiten Knochenteil vor, um welchen die Fehlstellung korrigiert werden kann.

Ein Aspekt der Erfindung bezieht sich auf ein Set von zwei oder mehr der obengenannten Schablonen, wobei die Fixierungsführungen und die Korrekturführungen der Schablonen für unterschiedliche Korrekturwinkel geeignet sind.

In einer bevorzugten Ausführungsform weist die Schablone eine, zwei oder mehr Fixierungsvorrichtungen auf, um den Korrekturteil relativ zum Basisteil zu fixieren. Die Fixierungsvorrichtung kann geeignet sein, den Korrekturteil relativ zum Basisteil in einer, zwei oder mehreren verschiedenen Positionen zu fixieren. Ein Beispiel für eine Fixierungsvorrichtung ist ein Stift, welcher gleichzeitig in Fixierungsbohrungen, die am Korrekturteil und am Basisteil angeordnet sind, eingeschoben werden kann. Andere Beispiele für Fixierungsvorrichtungen sind Klemmvorrichtungen, wie eine Klemmschraube, Rastvorrichtungen und formschlüssige Aufnahmen oder mehrere Schrauben.

Der Basisteil und der Korrekturteil sind bevorzugt lösbar durch die Fixiervorrichtung zueinander fixierbar. Durch die Fixierung kann ein Korrekturwinkel für die Osteotomie voreingestellt werden.

In einer bevorzugten Ausführungsform sind der Korrekturteil und der Basisteil beweglich miteinander verbunden. Dadurch wird eine Handhabung der Schablone vereinfacht. In einer bevorzugten Ausführungsform sind der Korrekturteil und der Basisteil so beweglich miteinander verbunden, dass verschiedene Korrekturwinkel eingestellt werden können.

In einer bevorzugten Ausführungsform wird der Korrekturteil durch eine Schiene oder einen Schlitz oder einen Rand der Schablone geführt.

Ein weiterer Aspekt der Erfindung betrifft eine Schablone für eine Domosteotomie mit einem Basisteil und einem Osteotomieteil. In einer bevorzugten Ausführungsform ist der Osteotomieteil beweglich mit dem Basisteil verbunden, wobei der Osteotomieteil zumindest eine Osteotomieführung für eine Domosteotomie zwischen einem ersten und einem zweiten Knochenteil aufweist. Bevorzugt ist der Osteotomieteil so beweglich mit dem Basisteil verbunden oder vebindbar, dass die Osteotomieführung entlang einer gekrümmten Strecke bewegbar ist. Die gekrümmte Strecke kann ein Kreisbogensegment sein. Dadurch kann eine Domosteotomie durchgeführt werden. Die Schablone muss nicht speziell für einen Knochen ausgewählt werden wie im Stand der Technik, sondern ist gleichzeitig für verschiedene Knochengrössen verwendbar.

Bevorzugt ist die gekrümmte Strecke kreisbogenförmig entlang eines vorbestimmten Radius.

Der Basisteil und der Osteotomieteil sind bevorzugt miteinander an einem Drehpunkt gelenkig verbunden. Besonders bevorzugt ist der Osteotomieteil schwenkbar mit dem Basisteil verbunden. Dadurch kann die Osteotomieführung entlang eines vorgesehenen Schnittes geführt werden. Der Osteotomieteil kann als Pendel ausgebildet sein.

Der Osteotomieteil kann um den Drehpunkt drehbar angeordnet sein. Der Osteotomieteil kann an dem Drehpunkt mit dem Basisteil verbunden sein. Dadurch kann die Osteotomieführung entlang eines Kreisbogenabschnittes geführt werden. Dies hat den Vorteil, dass eine kreisbogenförmige Domosteotomie durchgeführt werden kann.

Der Osteotomieteil kann stufenlos oder in diskreten Schritten schwenkbar sein.

In einer bevorzugten Ausführungsform ist der Osteotomieteil mit der Osteotomieführung entlang einer vordefinierten Strecke bewegbar. Die vordefinierte Strecke ist bevorzugt gekrümmt, besonders bevorzugt kreisbogenförmig.

In einer bevorzugten Ausführungsform weist der Basisteil eine bogenförmige Ausnehmung auf. Die Osteotomieführung ist bevorzugt entlang der bogenförmigen Ausnehmung bewegbar. Dadurch kann der Osteotomieteil auf dem Basisteil angeordnet werden und einfach gehandhabt werden.

In einer bevorzugten Ausführungsform weist die Osteotomieführung zusätzlich eine Bohrhilfe auf. Die Bohrhilfe ist insbesondere eine Bohrhülse. Dadurch kann ein Bohrer geführt werden. Die Fixierungsführung und/oder die Korrekturführung können eine Bohrhülse aufweisen.

In einer bevorzugten Ausführungsform weist die Schablone eine erste Seite für einen Knochen ("Knochenseite") und eine zweite einem Benutzer zugewandte Seite ("Benutzerseite") auf.

Ein weiterer Aspekt der Erfindung betrifft eine Schablone, wobei die erste Seite an einen Knochen eines Patienten angelegt werden kann. Die erste Seite kann an eine individuelle Knochenform angepasst sein oder anpassbar sein. Alternativ kann die Schablone auch an einen generischen Patienten angepasst sein. Dadurch kann die Schablone einfach an einen Knochen eines Patienten angelegt werden und präzise ausgerichtet werden.

In einer bevorzugten Ausführungsform weist die Schablone eine Schnittstelle für eine Passform für einen Knochen auf. Dadurch kann, beispielsweise in einem additiven Fertigungsverfahren, eine an den Patienten angepasste Passform gefertigt werden und dann mit der Schablone verbunden werden. In der Folge kann die Schablone wiederverwendet werden und ein genaues Anlegen an den Knochen sichergestellt werden.

Eine weitere erfindungsgemässe Variante betrifft ein Set aus einer Knochenplatte mit zwei oder mehr Plattenlöchern und einer oben beschriebenen Schablone.

Ein weiterer erfindungsgemässer Aspekt betrifft ein Set, welches eine Schablone wie oben beschrieben enthält und eine Ausrichthilfe zur initialen Ausrichtung der Schablone an einem Knochen. Die Ausrichthilfe kann länglich ausgebildet sein. Die Ausrichthilfe kann ein Stab sein. Die Ausrichthilfe kann an der Schablone fixierbar sein. Der Ausrichthilfe kann insbesondere am Korrekturteil der Schablone fixierbar sein. Durch die Ausrichthilfe kann der Korrekturteil präziser ausgerichtet werden.

Ein weiterer Aspekt der Findung betrifft ein Verfahren zur Vorbereitung einer Fixation durch eine Osteosynthese. In dem Verfahren wird eine Schablone, bevorzugt eine Schablone wie oben beschrieben, bereitgestellt. Ein Korrekturteil der Schablone wird relativ zu einem Basisteil der Schablone anhand eines vorbestimmten Korrekturwinkels positioniert und die Teile werden anschliessend zueinander fixiert. Der Basisteil wird mit zumindest einem Knochenfixierungselement an einem Knochen eines Patienten fixiert. Eine definierte Anzahl von Löchern wird in einem ersten Teil des Knochens mit einem Schneidwerkzeug geschnitten. Die Löcher werden mit jeweils einer Fixierungsführung auf dem Basisteil der Schablone geschnitten. Weiterhin wird eine definierte Anzahl von Löchern in einem zweiten Teil des Knochens mit einem Schneidwerkzeug geschnitten. Dabei wird das Schneidwerkzeug für jedes Loch durch je eine Korrekturführung geführt.

In einer bevorzugten Ausführungsform werden im ersten und/oder im zweiten Teil zwei, drei oder mehr Löcher geschnitten. Bevorzugt werden die Löcher gebohrt.

Der Korrekturwinkel eines Knochens kann aufgrund von bildgebenden Verfahren wie Röntgen oder CT, im Vornerein bestimmt werden.

Bevorzugt wird anschliessend ein Schneidwerkzeug in eine Führung auf dem Osteotomieteil eingeführt und ein erstes Loch in den Knochen einer ersten Stelle geschnitten. Anschliessend wird der Osteotomieteil relativ zum Basisteil entlang einer vordefinierten Strecke verschoben. Bevorzugt wird der Osteotomieteil entlang eines Kreisbogens verschoben. Dann wird ein zweites Loch an einer zweiten Stelle in den Knochen geschnitten. Optional können weitere Löcher (dritte, vierte, fünfte, usw.) geschnitten werden. Im nächsten Schritt wird der Knochen durchtrennt. Der Knochen kann beispielsweise mittels eines Meissels durchtrennt werden. Alternativ kann der Knochen auch mit einem Laser, einer oszillierenden Säge, insbesondere mit einem gewölbten Sägeblatt, einer Piezosäge, einer Fräse oder einem anderen Trennverfahren durchtrennt werden.

Eine bevorzugte Anwendung für das oben genannte Verfahren und die oben genannte Schablone ist eine Sprunggelenks-Umstellungsosteotomie, bei welcher eine Achsenfehlstellung am Rückfuss korrigiert wird. Zusätzlich kann die Schablone auch eingesetzt werden für insbesondere die folgenden Osteotomien: Rekonstruktion von Unterkiefern, Knieoperation, Kalkaneusosteotomien, Handgelenk- oder Ellbogenoperationen oder Humerusosteotomien.

Anhand von Figuren, welche lediglich Ausführungsbeispiele darstellen, wird die Erfindung im Folgenden näher erläutert. Es zeigen schematisch:
- Fig. 1-6:: verschiedene bekannte Keilosteotomien,
- Fig.7:: eine erfindungsgemässe Schablone für eine Osteotomie,
- Fig. 8A:: einen Abschnitt der Schablone aus Figur 7,
- Fig. 8B und C:: Visualisierungen der Einstellung der Korrekturwinkel,
- Fig. 9A:: einen Ausschnitt des Sprunggelenkes,
- Fig. 9B bis 11:: den schematischen Ablauf einer Domosteotomie an einem Sprunggelenk mit der Schablone gemäss Figur 4,
- Fig. 12A und 12B:: Ansichten der Schablone aus Figur 7 mit einer Ausrichthilfe,
- Fig. 13:: eine Seitenansicht einer erfindungsgemässen Schablone für eine Domosteotomie,
- Fig. 14:: eine erste alternative erfindungsgemässe Ausführungsform für eine Schablone für eine Domosteotomie,
- Fig. 15:: eine zweite alternative erfindungsgemässe Ausführungsform für eine Schablone für eine Domosteotomie,
- Fig. 16:: eine dritte alternative erfindungsgemässe Ausführungsform für eine Schablone für eine Domosteotomie und
- Fig. 17:: eine Osteosyntheseplatte zur Verwendung mit der Schablone gemäss Figur 16.

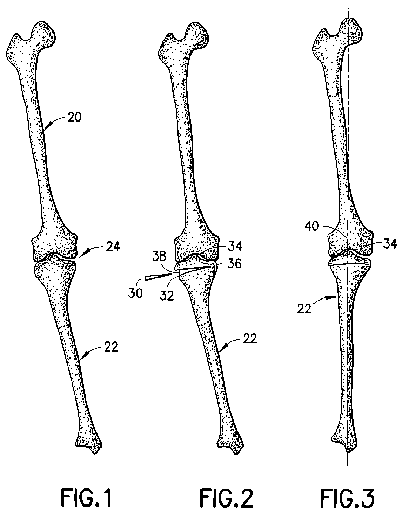

In den Figuren 1 bis 6 werden zwei verschiedene Keilosteotomien beschrieben, wie sie auf bekannte Art und Weise durchgeführt werden.

Unter Bezugnahme auf die Zeichnung ist in FIG. 1 eine typische Fehlausrichtung zwischen einem Oberschenkel 20 und einer Tibia 22 des Beines eines Patienten dargestellt, wobei die Fehlausrichtung auf ein Varusknie 24 zurückzuführen ist, das als Folge einer Arthrose im Knie im medialen Bereich aufgetreten ist. Die Korrektur der Fehlausrichtung unter Verwendung eines bekannten Verfahrens der hohen Schienbeinkeilosteotomie ist in den FIGS. 2 und 3 veranschaulicht. Die Figuren zeigen die Entfernung eines keilförmigen Abschnitts 30 des Knochens aus der proximalen Tibia, um einen Spalt 32 zu schaffen, der sich quer zur Längsachse der Tibia 22 erstreckt. Ein proximales Fragment 34 bleibt mit der proximalen Tibia an einem kortikalen Scharnier 36 verbunden. Der Spalt 32 ist keilförmig und beinhaltet einen Winkel 38, der vor dem Verfahren berechnet wurde, um die gewünschte Korrektur durchzuführen. Wie in FIG. 3 zu sehen ist, wird die Korrektur erreicht, indem die Lücke 32 geschlossen wird, um das proximale Fragment 34 wieder mit der proximalen Tibia zu verbinden, wodurch die Tibia 22 neu ausgerichtet wird, um die Tibia 22 in Ausrichtung mit der Achse 40 des Beines des Patienten zu bringen. Wie in Figuren 1-3 zu sehen ist, wird ein Fragment des Knochens entnommen. Diese Art der Neuausrichtung ist häufig ungenau und die Genauigkeit des Korrekturwinkels hängt in erheblichem Masse von der Geschicklichkeit eines Operateurs ab. Ausserdem benötigt das Gewebe des Knochens einige Zeit, um wieder zusammen zu wachsen.

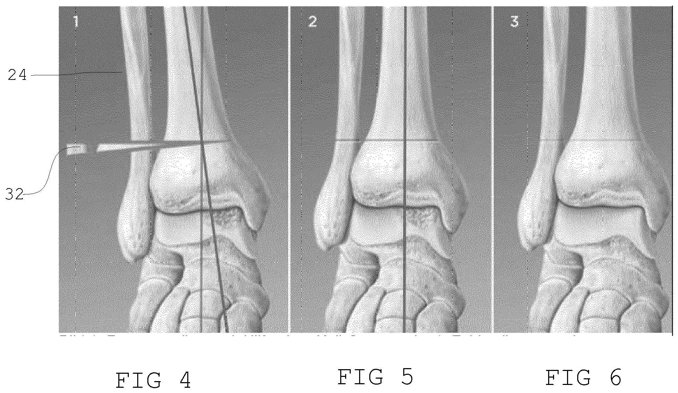

Eine weitere Fehlausrichtung mit einer bekannten Keilosteotomie ist in den Figuren 4-6 gezeigt. Infolge einer Arthrose im Fussgelenk ist eine Varusstellung 24 entstanden. Die Fehlausrichtung kann in einem bekannten Verfahren korrigiert werden, indem oberhalb des Fussgelenkes an Schienbein und Wadenbein ein keilförmiger Abschnitt des Knochens entfernt wird, um einen Spalt 32 zu schaffen, der sich quer zur Längsachse des Schienbeins und des Wadenbeins erstreckt. Wie oben beschrieben, bestimmt der Winkel des Keils einen Korrekturwinkel. Der Korrekturwinkel wird vor der Operation mithilfe von Röntgenbildern berechnet und ein Chirurg versucht während der Operation, einen Keil mit genau dem vorgegebenen Winkel zu entfernen. Nach dem Entfernen des Knochenteils werden die Knochen aufeinander geklappt, und mithilfe einer Osteosyntheseplatte zueinander fixiert. Nach der Operation nimmt die Osteosyntheseplatte zumindest einen Teil der Belastungen auf.

Es zeigt sich, dass der Korrekturgrad von der sorgfältigen Berechnung des Winkels des keilförmigen Spaltes 32 vor dem Eingriff abhängt und der Chirurg wenig Gelegenheit hat, während des Eingriffs Korrekturen vorzunehmen. Eine Fehlanpassung des Knochens am geschlossenen Spalt 32 kann sowohl zu kurzfristigen Heilungsproblemen als auch zu langfristigen Problemen, wie z.B. Drift, führen. Der Knochen geht teilweise verloren, da der keilförmige Teil des Knochens entfernt werden muss. Die transversale Ausrichtung der Schnitte, die für die Bildung des keilförmigen Teils des Knochens erforderlich sind, erfordert einen relativ schwierigen Zugang durch das umgebende Weichgewebe mit der Möglichkeit schädlicher Auswirkungen.

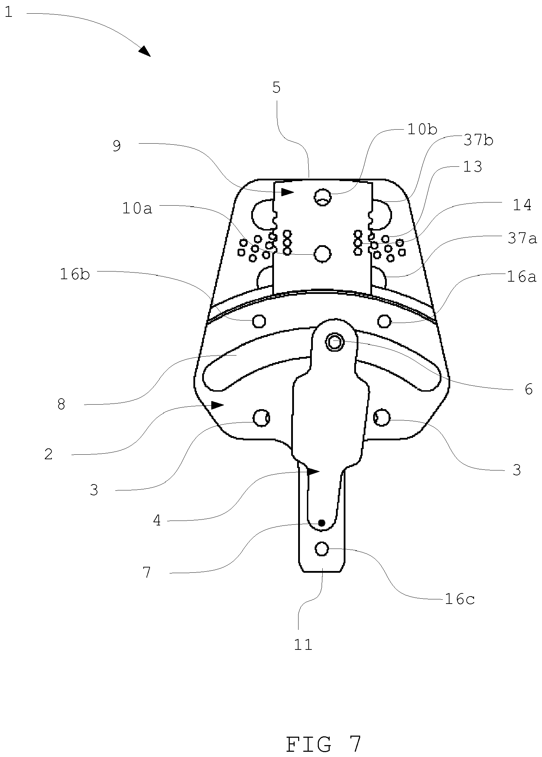

Figuren 7 bis 8B zeigen eine Schablone 1 gemäss der Erfindung, mit der eine Domosteotomie durchgeführt werden kann. Mit der Schablone 1 können die Nachteile der oben beschriebenen Keilosteotomie überwunden werden. Die Schablone ist aus drei Teilen - nämlich einem Basisteil 2, einem Korrekturteil 9 und einem Osteotomieteil 4 - aufgebaut und weist ein proximales Ende 5 und ein distales Ende 11 auf. An dem Basisteil 2 sind der Osteotomieteil 4 und der Korrekturteil 9 befestigt. Der Osteotomieteil 4 und der Korrekturteil 9 sind jeweils beweglich mit dem Basisteil 2 verbunden. Der Basisteil 2 weist ein längliches Loch 8 auf, welches einer vordefinierten Strecke folgt. Das längliche Loch 8 ist gebogen entlang eines Kreisbogensegmentes. Der Osteotomieteil 4 ist an einem ersten Ende an einem Drehpunkt 7 drehbar mit dem Basisteil 2 verbunden. An einem zweiten Ende des Osteotomieteils 4 ist eine Osteotomieführung 6 angeordnet. Die Osteotomieführung 6 ist schwenkbar um den Drehpunkt schwenkbar, sodass sie dem länglichen Loch 8 folgt.

Die Schablone 1 wird durch Aufnahmen 16 (16a, 16b, 16c) an einem Knochen fixiert. Durch die Aufnahmen 16a, 16b kann ein Kirschnerdraht geführt werden, mit dem die Schablone am Knochen fixiert wird. Die Aufnahmen 16a, 16b sind mittig zwischen dem proximalen Ende 5 und dem distalen Ende 11 angeordnet. Eine dritte Aufnahme 16c ist im Bereich des distalen Endes 11 angeordnet.

Ausserdem weist die Schablone 1 in einem distalen Bereich zwei Fixierungsführungen 3 auf, die als Bohrführungen ausgeführt sind. Die Fixierungsführungen 3 führen einen Bohrer entlang ihrer Achse. Dadurch kann ein Bohrer eine Plattenlochposition festlegen.

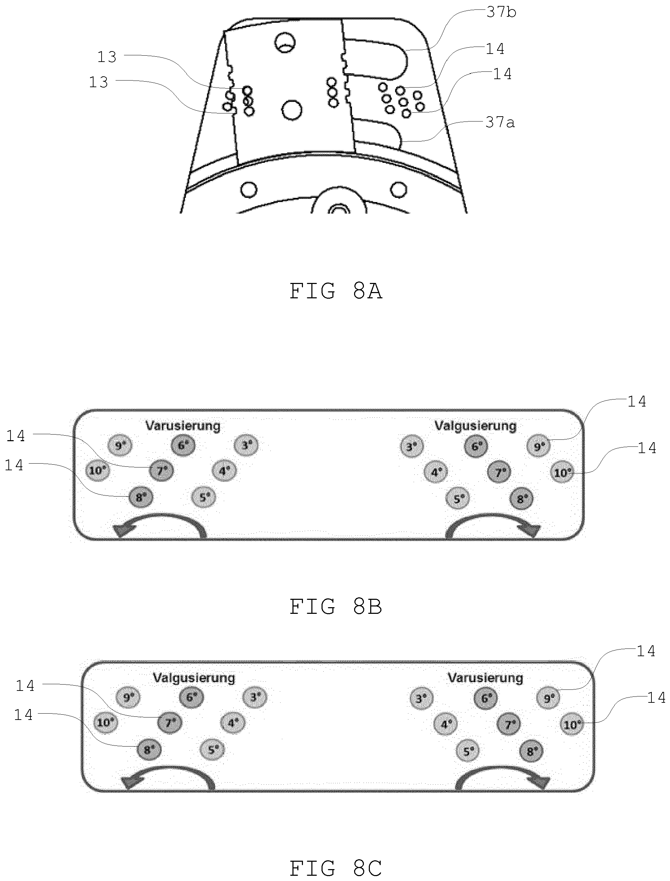

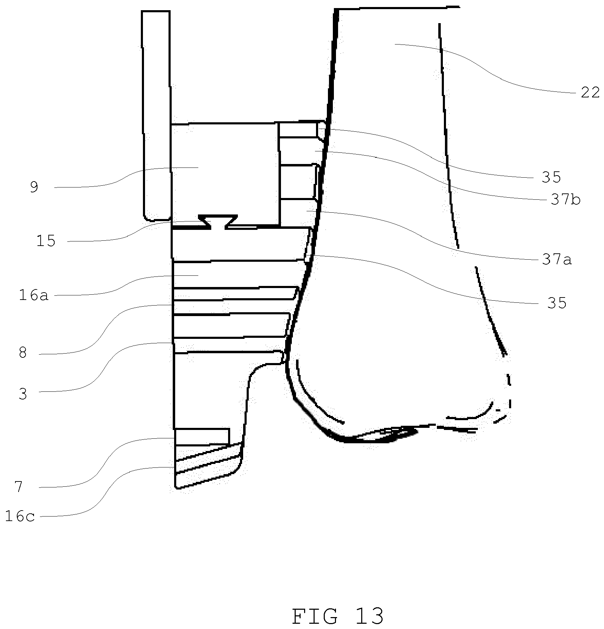

In einem proximalen Bereich des Basisteils 2 ist der Korrekturteil 9 beweglich mit dem Basisteil 2 verbunden. Der Korrekturteil ist auf einer Schiene 15 (siehe Figur 13) angeordnet. Der Korrekturteil 9 umfasst eine Führung für die Schiene 15 und weist passende, als Stiftlöcher ausgebildete Fixierungsvorrichtungen 14 auf. Der Korrekturteil 9 kann auf der Schiene 15 entlang eines Kreisbogens verschoben werden, bis ein vorbestimmter Winkel erreicht wird (siehe Figur 8A). In dieser Position wird der Korrekturteil 9 fixiert, indem ein Stift durch die Fixierungsvorrichtungen 13, 14 geschoben wird.

Zudem weist der Korrekturteil zwei Korrekturführungen 10a und 10b auf. Der Basisteil 2 weist in seinem proximalen Teil zwei weitere, bevorzugt längliche und gekrümmte, Ausnehmungen 37a, 37b auf. Die Ausnehmungen erstrecken sich entlang der möglichen Positionen der Korrekturführungen 10a, 10b, sodass durch die Korrekturführungen 10a, 10b und die Ausnehmungen 37a, 37b mit einem Bohrer ein Loch in einem Knochen eingebracht werden kann.

An dem Basisteil 2 sind ebenfalls Fixierungsvorrichtungen 13 angeordnet, die als Stiftlöcher ausgebildet sind. Die Fixierungsvorrichtungen 13 sind zwischen den Ausnehmungen 37a, 37b angeordnet.

Die Löcher der Fixierungsvorrichtungen 13 sind in mehreren (drei) parallel verlaufenden Reihen angeordnet. Dabei sind die Löcher der Reihen zueinander versetzt, sodass der Winkel fein eingestellt werden kann (siehe Figur 8B und 8C).

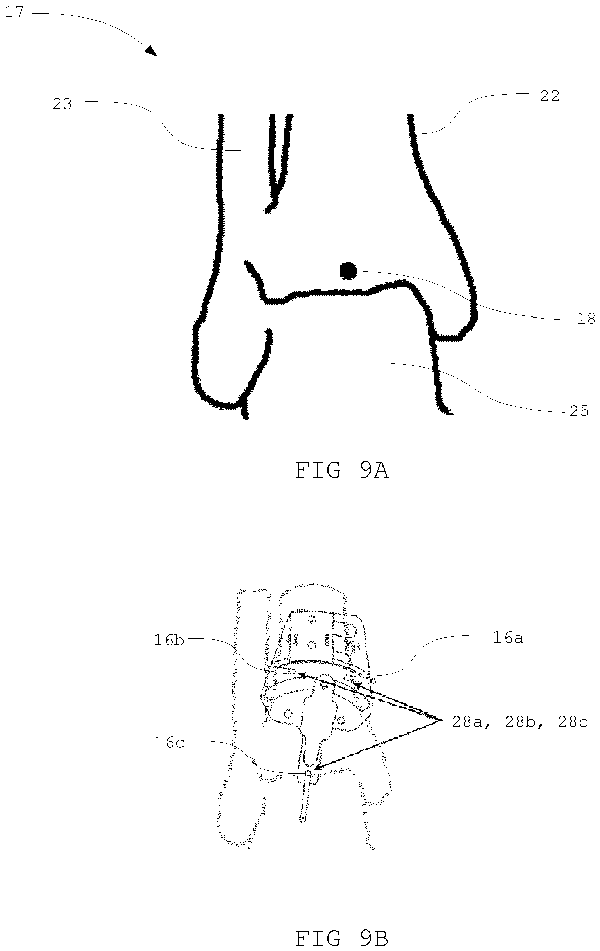

Eine Verwendung der oben beschriebenen Schablone wird im Folgenden im Zusammenhang mit den Figuren 9-11 beschrieben. In einer Domosteotomie mit der oben beschriebenen Schablone wird zunächst anhand von Röntgenbildern des Fusses (bis zum Knie) eine Achsenfehlstellung im oberen Sprunggelenk ermittelt. Die Achsenfehlstellung kann in der Koronarebene auswärts (Varus) oder einwärts (Valgus)und in der Sagittalebene nach vorne (antkurvatum), oder nach hinten (rekurvatum) oder eine Kombination der beiden sein.

Ein Winkel der Achsenfehlstellung wird ermittelt, indem der Winkel auf den präoperativ angefertigten Röntgenbildern oder Schnittbildern ausgemessen und entweder mit einem gesunden Kollektiv verglichen oder die Gegenseite als Referenz verwendet wird.

Der ermittelte Korrekturwinkel wird dann durch Verschieben des Korrekturteiles an der Schablone 1 eingestellt. Wie in Figuren 8B (für das rechte Bein) und in Figur 8C (linkes Bein) gezeigt, entspricht jede der Fixierungsvorrichtungen 14 auf dem Basisteil einem Korrekturwinkel. Der Korrekturteil wird relativ zum Basisteil in den gewünschten Korrekturwinkel verschoben und anschliessend werden die Fixierungsvorrichtungen 13, 14 zueinander mittels eines Stiftes fixiert.

Figur 9A zeigt schematisch ein oberes Fussgelenk 17. Das obere Fussgelenk wird gebildet durch die distalen Enden des Schienbeins 22 und des Wadenbeins 23 sowie durch das proximale Ende des Sprungbeins 25. Während der Operation wird zunächst das obere Fussgelenk 17 freigelegt. Als erstes wird ein Rotationszentrum für die Domosteotomie bestimmt. Das Rotationszentrum ist eine erste Verankerungsposition 18. Idealerweise wird die erste Verankerungsposition 18 an einem distalen Ende des Schienbeins (Tibia) markiert.

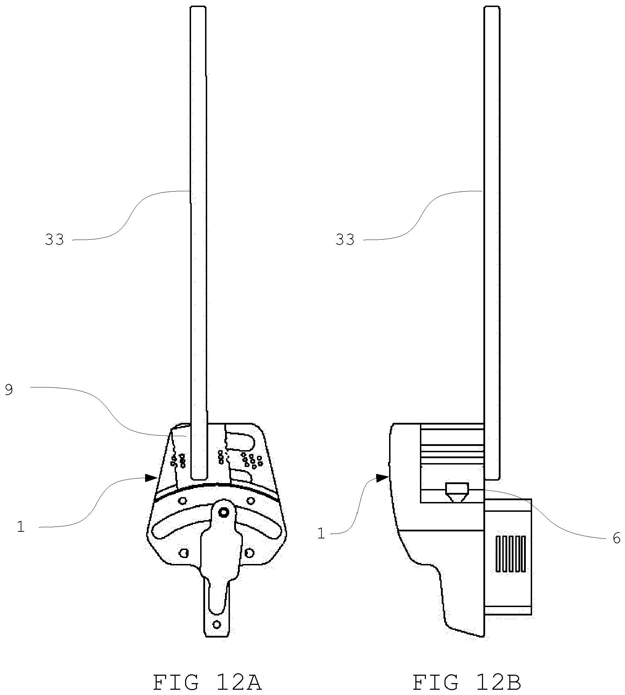

Die Aufnahme 16c am distalen Ende 11 der Schablone 1 wird dann mit einem Kirschnerdraht 28a (K-Wire) am Schienbein in der Verankerungsposition 18 fixiert (siehe Figur 9B). In dieser Position kann die Schablone 1 als Ganzes relativ zum Schienbein 22 geschwenkt werden. Eine dem Knochen zugewandte Seite der Schablone 1 (siehe Figur 13) ahmt eine Form des freigelegten Schienbeins im distalen Bereich des Basisteils 2 nach, sodass die Schablone einfach angelegt werden kann. Dabei wird die Schablone mithilfe eines Ausrichtungsstabes 33 entlang der Tibia angelegt (siehe Figuren 12A und 12B). Durch den Ausrichtungsstab 33 können die Korrekturführungen 10a, 10b auf dem Korrekturteil 9 mittig auf dem Schienbein 22 ausgerichtet werden.

Anschliessend wird die Schablone 1 mit zwei weiteren Kirschnerdrähten an den Aufnahmen 16a und 16b vollständig am Schienbein 22 fixiert (siehe Figur 9B). In dieser Position ist die Schablone 1 fix und unbeweglich mit dem Schienbein 22 verbunden.

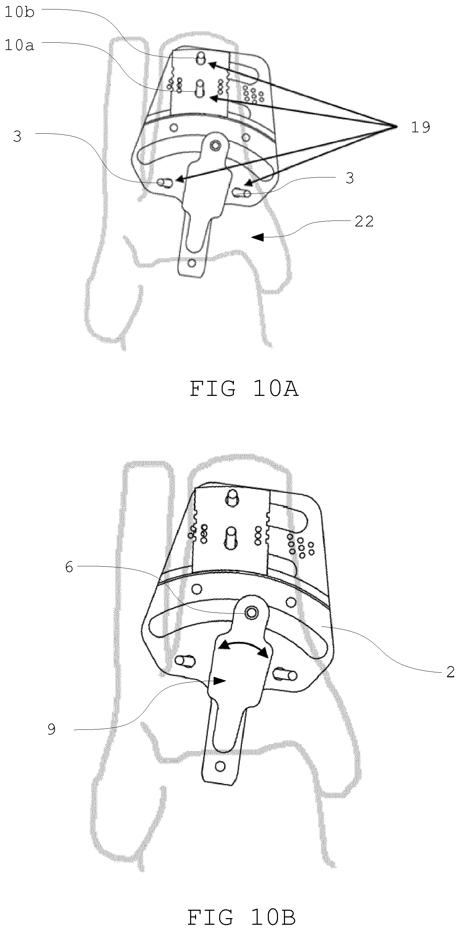

Im nächsten Schritt (siehe Figur 10A) werden mit einem Bohrer 19 Plattenlöcher für eine Osteosyntheseplatte in das Schienbein gebohrt. Als Hilfe für die senkrechte Ausrichtung der Bohrungen und für die genaue Positionierung sind die Korrekturführungen 10a, 10b und die Fixierungsführungen 3 vorgesehen. Mithilfe von diesen Führungen und dem Bohrer 19 (schematisch als Bohrspitze in Figur 10A gezeigt) werden Plattenlöcher in das Schienbein 22 gebohrt. Diese Bohrungen können beispielsweise mit einem 2.7 mm Bohrer angebracht werden.

Da die Korrekturführungen auf dem Korrekturteil um den Korrekturwinkel verschoben wurden, werden die Bohrungen distal und proximal von der Osteotomie genau zueinander positioniert.

Im nächsten Schritt wird die Osteotomie durchgeführt. Wie oben beschrieben, ist der Osteotomieteil 9 schwenkbar zum Basisteil 2 angeordnet. Der Osteotomieteil 9 wird zunächst in eine erste Position gebracht (siehe Figur 10B) und ein erstes Loch wird mithilfe der Osteotomieführung 6 in das Schienbein 22 gebohrt. Anschliessend wird der Osteotomieteil 9 um einen Winkelbetrag verschoben, sodass in regelmässigen Abständen Bohrungen im Schienbein vorgesehen werden.

Für die Osteotomie ist es vorteilhaft, so wenig Knochenmaterial wie möglich durch die Trennung zu entfernen. Daher kann für die Osteotomiebohrungen ein Bohrer mit einem kleineren Durchmesser verwendet werden, bzw. eine Führung mit einem kleineren Durchmesser, beispielsweise ein Bohrer mit einem Durchmesser von 2 mm. Dies ermöglicht auch, die Bohrungen näher beieinander zu legen, was das spätere Meisseln vereinfacht. Wenn alle Bohrungen für die Osteotomie durchgeführt sind, kann die Schablone 1 wieder entfernt werden. Dafür werden die Kirschnerdrähte entfernt und die Schablone beiseitegelegt.

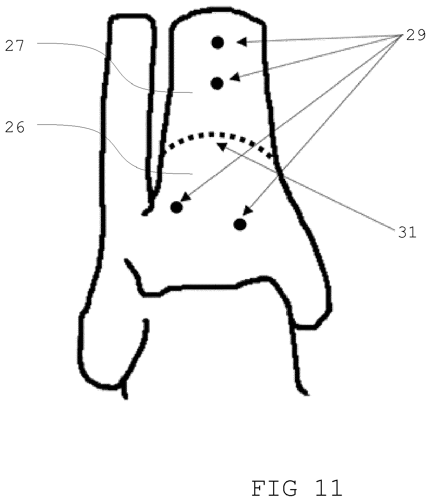

Der Zustand des Knochens, nachdem alle Bohrungen durchgeführt worden sind, ist in Figur 11 gezeigt. Die Perforationen 31 für die Osteotomie werden dann genutzt, indem mit einem Hammer und einem Meissel die Stege zwischen den Bohrungen für die Osteotomie durchbrochen werden.

Anschliessend werden die beiden Teile der Tibia (der erste distale Teil 26 und der zweite proximale Teil 27) durch den Chirurgen zueinander verdreht, sodass sie etwa um den gewünschten Korrekturwinkel zueinander verschoben werden. Eine Osteosyntheseplatte wird aufgelegt und mittels Knochenschrauben in den Bohrungen 29 für die Osteosyntheseplatte fixiert. Zunächst wird die Platte an den distalen Bohrungen, die mit den Fixierungsführungen 3 gebohrt wurden, fixiert. Anschliessend kann der zweite Knochenteil mit den proximalen Bohrungen, welche mit den Korrekturführungen gebohrt wurden, fixiert werden. So kann die Osteosyntheseplatte als Hilfsmittel zum Verschieben der Teile des Schienbeins zueinander verwendet werden. Durch die Knochenschrauben und die Osteosyntheseplatte werden der erste und der zweite Teil des Schienbeins in den zuvor festgelegten Korrekturwinkel gebracht und exakt zueinander fixiert.

Wie in Figur 13 zu sehen ist, weist die Schablone an ihrer dem Knochen zugewandten Seite ein dem Schienbein 22 angepasstes Profil 35 auf. Das Profil wird durch eine imaginäre Linie gebildet, die einer typischen Schienbeinkontur eines erwachsenen Menschen entspricht. Das Loch 8, die Aufnahme 16a, die Fixierungsführung 3 und die Ausnehmungen 37a, 37b unterbrechen dabei das Profil. Durch das angepasste Profil können Druckstellen und unerwünschte Spannungen vermieden werden.

Eine Längsachse der Aufnahme 16c für das Rotationszentrum ist geneigt. Dadurch kann ein Rotationszentrum auf einem Knochen markiert werden und die Schablone dann so angelegt werden, dass der Drehpunkt 7 zum angezeichneten Rotationszentrum ausgerichtet ist (insb. deckungsgleich). In anderen Varianten können, abhängig vom Neigungswinkel (z.B. grösser als 10°), Rotationszentren gewählt werden, die ausserhalb vom Knochen liegen.

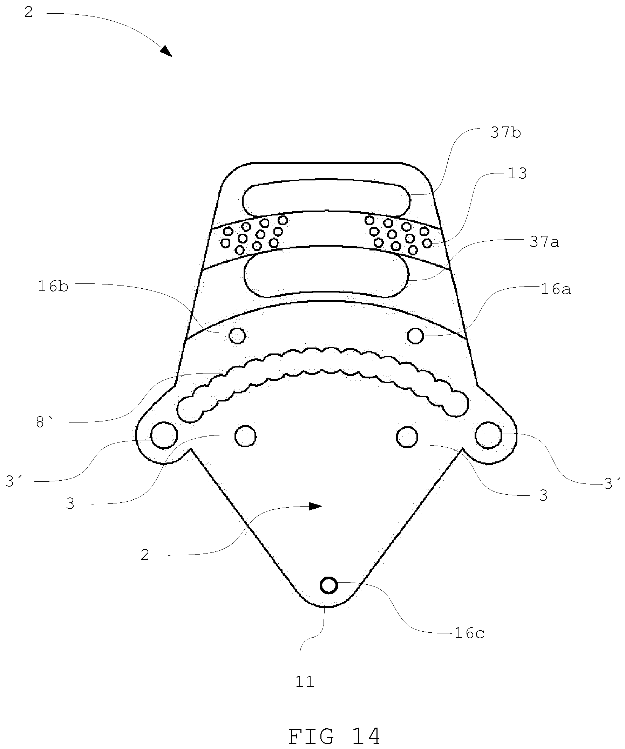

Figur 14 zeigt den Basisteil 2 einer ersten alternativen Ausführungsform einer Schablone 1. Im Unterschied zu der in Figur 7 gezeigten Ausführungsform weist das gekrümmte Loch 8 zusätzlich Riffelungen 8' auf. Durch die Riffelungen 8' werden diskrete Positionen für die Osteotomieführung vorgegeben. Alternativ kann die Schablone 1 auf dem Basisteil 2 auch eine Mehrzahl von Osteotomieführungen 6 aufweisen, die nebeneinander angeordnet sind.

Ein Rand des Loches 8' weist in regelmässigen Abständen Einkerbungen auf, die die Riffelungen 8' bilden. In Figur 14 sind die Abstände zwar regelmässig, aber es sind auch Einkerbungen in unregelmässigen Abständen denkbar. So können beispielsweise in Bereichen mit festem Knochenmaterial (z.B. die Knochenrinde) die Einkerbungen näher beieinanderliegen und in Bereichen mit weniger festem Knochenmaterial die Einkerbungen weiter auseinanderliegen.

Der in Figur 14 gezeigte Basisteil 2 weist ausserdem zwei zusätzliche Fixierungsführungen 3' auf. Durch die zusätzlichen Fixierungsführungen 3' kann es einfacher sein, den Basisteil 2 auf einem Knochen zu fixieren. Dies ist insbesondere der Fall, wenn die Fixierung durch die Fixierungsführungen 3 nicht ausreicht oder nicht möglich ist. Dies kann insbesondere bei grossen Korrekturwinkeln der Fall sein, wenn der Basisteil schräg auf dem Knochen anliegt. In bestimmten Ausführungsformen, wie beispielsweise in Figur 14 gezeigt, können die zusätzlichen Fixierungsführungen 3' auf sich vom Basisteil erstreckenden Lappen liegen.

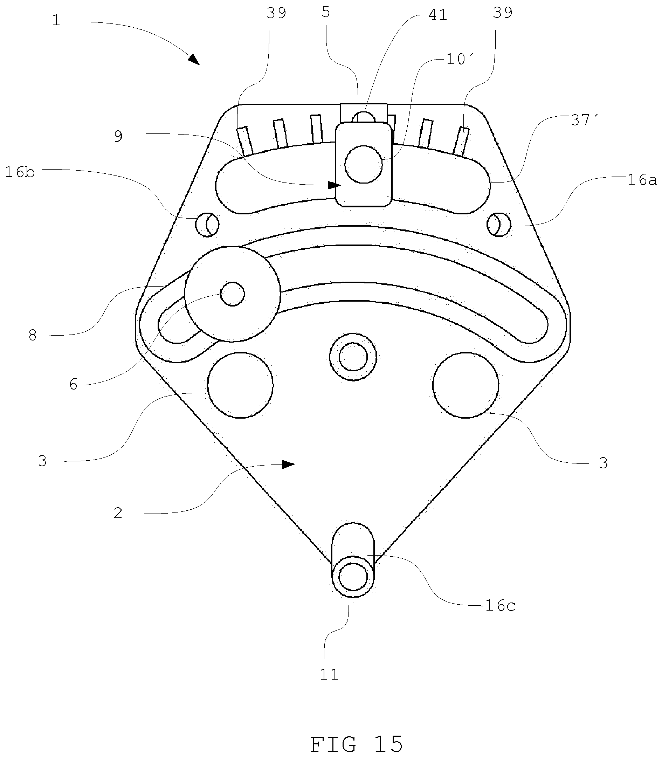

Figur 15 zeigt eine zweite alternative Ausführungsform für die Schablone 1. Im Unterschied zu der in Figur 7 gezeigten Ausführungsform ist die Fixierungsvorrichtung nicht als Stiftführung ausgebildet. Die Fixierungsvorrichtung wird in der Ausführungsform durch eine Feststellschraube gebildet. Der Korrekturteil 9 wird dabei mittels der Schraube (nicht gezeigt) angezogen und an dem Basisteil 2 festgeklemmt. Der schwenkbare Korrekturteil 9 weist eine einzelne Korrekturführung 10' auf. Alternativ dazu kann ein Anschlagelement (nicht gezeigt) an dem Gewinde der Schraube angeordnet sein, sodass der Basisteil 2 beim Anziehen der Schraube zwischen Anschlagelement und Schraubenkopf eingeklemmt wird und so den Korrekturteil 9 am Basisteil 3 fixiert. In der Variante von Figur 15 sind die Aufnahmen 16b und 16a schräg. Dadurch kann die Schablone 1 auch an kleineren Knochen fixiert werden. Bevorzugt konvergieren K-Drähte, die durch die Aufnahmen 16a und 16b eingeführt werden.

Der Korrekturteil 9 weist ausserdem ein Fenster 41 auf, mittels dem eine Winkelanzeige 39 abgelesen werden kann. Um einen bestimmten Korrekturwinkel einzustellen, wird der Korrekturteil 9 so lange verschoben, bis der gewünschte Winkel in dem Fenster 41 erscheint. Dann wird die Schraube angezogen.

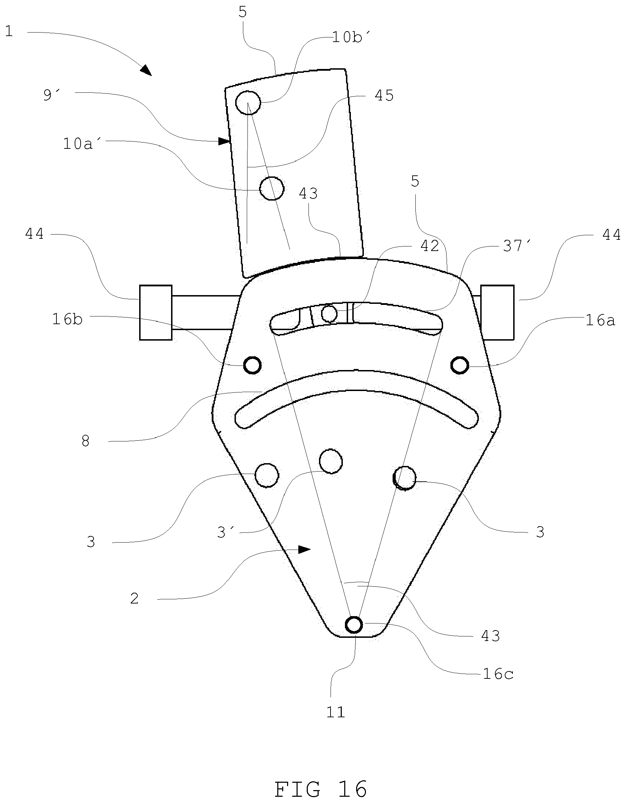

Eine dritte alternative Ausführungsform der Schablone 1 wird in Figur 16 gezeigt. Die dritte alternative Ausführungsform weist eine zusätzliche dritte Fixierungsführung 3' auf. Weiterhin unterscheidet sich die dritte alternative Ausführungsform von der in Figur 7 gezeigten Schablone 1 durch einen Korrekturteil 9'. Der Korrekturteil 9' erstreckt sich entlang der dem Knochen des Patienten zugewandten Seite. Das proximale Ende 5 des Basisteils 2 wird an und entlang einer Gegenfläche 43 des Korrekturteiles geführt. Die Gegenfläche 43 kann, wie in Figur 16 gezeigt, als Stufe ausgebildet sein. Von der Stufe erstreckt sich länglich ein Fortsatz, auf welchem ein Pin 42 angeordnet ist. Der Pin 42 wird in einer gekrümmten Ausnehmung 37' (insb. einem gebogenen Langloch) geführt. So kann der Korrekturteil entlang eines Winkelbereiches geschwenkt werden und auf den vorbestimmten Korrekturwinkel eingestellt werden.

Zusätzlich weist die Schablone 1 gemäß der dritten alternativen Ausführungsform Schrauben 44 auf. Die Schrauben 44 werden durch ein Gewinde im Basisteil 2 geführt und klemmen an ihrem Ende den Korrekturteil 9' ein. So erlauben die Schrauben eine stufenlose Einstellung Fixierung des Korrekturteils 9' in Relation zum Basisteil 2.

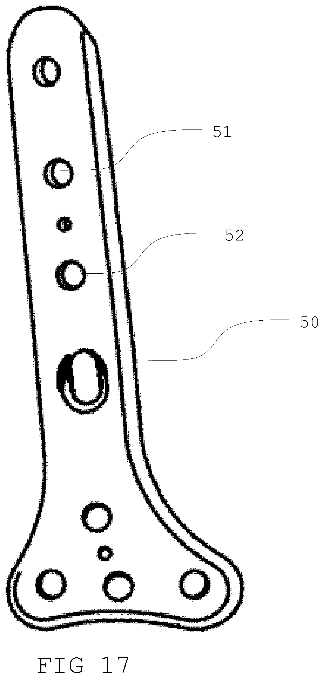

In dem in Figur 16 gezeigten Beispiel ist die Variante der Schablone für den rechten Fuss in 0° Neutralstellung gezeigt. Die zugehörige Platte 50 (siehe Fig. 17) ist nicht gerade, sondern leicht asymmetrisch. Deshalb sind die Korrekturführungen 10a', 10b' seitlich zueinander versetzt, so dass sie der Anordnung von Plattenlöchern 51, 52 entsprechen.

In den Ausführungsformen der Figuren 14-16 ist der Osteotomieteil nicht explizit gezeigt. Der Osteotomieteil kann analog zu den Darstellungen in Fig. 7 oder 10B ausgebildet sein. Alternativ kann er auch als entlang des Lochs 8 verfahrbarer Schlitten ausgebildet sein (in Fig. 15 angedeutet).Fig. 17 zeigt schematisch eine Osteosyntheseplatte 50 mit Plattenlöchern 51, 52 zur Verwendung mit einer Schablone 1 mit Korrekturführungen 10a', 10b', wie sie in Fig. 16 gezeigt ist.

## Patentansprüche

1. Schablone (1) für eine Domosteotomie aufweisend:
- einen Basisteil (2) mit zumindest einer Aufnahme (16) für ein Knochenfixierungselement zum Fixieren des Basisteils (2) an einem ersten Knochenteil, wobei der Basisteil (2) zumindest eine Fixierungsführung (33) zur Festlegung einer Plattenlochposition an dem ersten Knochenteil aufweist,
- einen Korrekturteil (9), wobei der Korrekturteil (9) zumindest eine Korrekturführung (10) zur Festlegung einer weiteren Plattenlochposition an einem zweiten Knochenteil aufweist,
- einen Osteotomieteil (4) mit zumindest einer Osteotomieführung (6) für eine Domosteotomie zwischen dem ersten und dem zweiten Knochenteil.

2. Schablone gemäss Anspruch 1, wobei die Fixierungsführung und die Korrekturführung derart angeordnet sind, dass die Plattenlochpositionen für die Knochenplatte die Korrektur einer Knochenfehlstellung erlauben.

3. Schablone gemäss Anspruch 1 oder 2, wobei der Korrekturteil (9) relativ zum Basisteil (2) in einer, zwei oder mehr Positionen fixierbar ist.

4. Schablone gemäss Anspruch 3, wobei die Schablone eine oder mehrere Fixierungsvorrichtungen (13, 14) aufweist, um den Korrekturteil (9) relativ zum Basisteil (2) zu fixieren.

5. Schablone gemäss einem der vorherigen Ansprüche, wobei der Korrekturteil (9) beweglich mit dem Basisteil (2) verbunden ist.

6. Schablone gemäss Anspruch 5, wobei der Korrekturteil (9) stufenlos zum Basisteil verstellbar ist und insbesondere durch eine Schiene (15) oder einen Schlitz oder einen Rand der Schablone geführt wird.

7. Schablone gemäss einem der vorherigen Ansprüche, wobei der Osteotomieteil (4) beweglich, insbesondere schwenkbar, mit dem Basisteil (2) verbunden ist.

8. Schablone gemäss Anspruch 7, wobei der Basisteil (2) und der Osteotomieteil (4) miteinander an einem Drehpunkt (7) gelenkig verbunden sind und Osteotomieteil (4) um den Drehpunkt drehbar angeordnet ist.

9. Schablone gemäss einem der vorherigen Ansprüche, wobei der Osteotomieteil (4) mit der Osteotomieführung (6) entlang einer vordefinierten Strecke (8) bewegbar ist, wobei die vordefinierte Strecke (8) bevorzugt gekrümmt ist, besonders bevorzugt kreisbogenförmig.

10. Schablone gemäss einem der vorherigen Ansprüche, wobei der Basisteil (2) eine bogenförmige Ausnehmung (8) aufweist, entlang welcher die Osteotomieführung (6) führbar ist.

11. Schablone gemäss einem der vorherigen Ansprüche, wobei die Osteotomieführung (6) zusätzlich eine Bohrhilfe aufweist.

12. Schablone gemäss einem der vorherigen Ansprüche, wobei die Schablone eine erste Seite für einen Knochen und eine zweite Seite aufweist wobei die erste Seite an eine individuelle Knochenform angepasst oder anpassbar ist oder einem gemittelten Knochen entspricht.
Set aus einer Knochenplatte enthaltend zwei oder mehr Plattenlöcher und einer Schablone gemäss einem der vorherigen Ansprüche.

13. Set enthaltend eine Schablone gemäss einem der vorherigen Ansprüche und eine Ausrichthilfe (33) zur initialen Ausrichtung der Schablone am Knochen.

14. Verfahren zum Vorbereiten einer Fixation durch eine Osteosynthese enthaltend die Schritte:
- Bereitstellen einer Schablone (1), bevorzugt einer Schablone gemäss einem der Ansprüche 1 bis 13,
- Positionieren und fixieren eines Korrekturteiles (9) der Schablone relativ zu einem Basisteil (2) der Schablone anhand eines vorbestimmten Korrekturwinkels
- Fixieren des Basisteils (2) mit zumindest einem Knochenfixierungselement (16) an einem Knochen
- Schneiden eines oder mehrerer Löcher für eine Knochenplatte in einen ersten Teil des Knochens mit einem Schneidwerkzeug, wobei je eine Fixierungsführung (3) auf dem Basisteil das Schneidwerkzeug führt,
- Schneiden eines oder mehrerer Löcher für die Knochenplatte in einem zweiten Teil des Knochens mit einem Schneidwerkzeug, wobei je eine Korrekturführung (10a, b) auf dem Korrekturteil (9) das Schneidwerkzeug führt.

15. Verfahren zur Durchführung einer Osteotomie enthaltend die Schritte gemäss Anspruch 14, wobei im ersten Teil und/oder im zweiten Teil zwei, drei oder mehr Löcher geschnitten, bevorzugt gebohrt werden.

16. Verfahren zur Durchführung einer Osteotomie enthaltend die Schritte gemäss Anspruch 14 oder 15 und:
- Einführen eines Schneidwerkzeuges in eine Führung auf einem Osteotomieteil (4),
- Schneiden eines ersten Loches im Knochen an einer ersten Stelle
- Schneiden eines zweiten Loches im Knochen an einer zweiten Stelle,
- Durchtrennen des Knochens, bevorzugt mittels eines Meissels.

17. Verfahren zur Durchführung einer Osteotomie enthaltend die Schritte gemäss Anspruch 16 und weiterhin den Schritt:
- Schneiden von weiteren Löchern, um den Knochen strukturell weiter zu schwächen.

18. Verfahren zur Durchführung einer Osteotomie enthaltend die Schritte gemäss Anspruch 16 oder 17 und zusätzlich enthaltend den Schritt:
- Verschieben des Osteotomieteils (4) relativ zum Basisteil (2) entlang einer vordefinierten Strecke, bevorzugt entlang eines Kreisbogens, wobei der Osteotomieteil (4) beweglich mit dem Basisteil (2) verbunden ist, um die Positionen der definierten Löcher festzulegen.
